# EUROPÄISCHE PATENTANMELDUNG

(11) **EP 4 186 587 A1**
(43) Veröffentlichungstag der Anmeldung: **31.05.2023**
(21) Anmeldenummer: 21210317.0
(22) Anmeldetag: 24.11.2021
(51) Int. Cl.: B01F 31/22, B01F 35/60, B01F 35/92, C12M 1/00, C12M 3/06

(54) **LABORSCHÜTTLER**

(71) Anmelder: Eppendorf SE, 22339 Hamburg (DE)
(72) Erfinder: SCHAFRINSKI, Arne, 22843 Bad Oldesloe (DE); FITZER, Jan, 22949 Ammersbek (DE)
(74) Vertreter: Kirchner, Christian

(57) **Zusammenfassung**

Die Erfindung betrifft Laborschüttler 1 zum Schütteln von in Probengefäßen gelagerten Laborproben in einer Kammer 2, die einen durch eine Kammeröffnung 3 ausziehbaren Auszugsmechanismus 10 zum Ausfahren einer die Probengefäße lagernden Probenplattform 14 in Auszugsrichtung A aufweist, der in einer eingefahrenen Position P1 schüttelbar ist und der vorzugsweise einen Sicherungsmechanismus 20 zum Sichern der Position P1 aufweist, mit einem schwenkbaren Griff 40, der in einer gesicherten Stellung S1 arretiert ist und dessen Entarretierung und eine in Ausziehrichtung A beginnende Schwenkbewegung die durch den Sicherungsmechanismus bewirkte Sicherung löst.

## Beschreibung

Die Erfindung betrifft Laborschüttler für das Schütteln von in Probengefäßen gelagerten Laborproben, insbesondere von Mikroorganismen in Suspension.

Temperaturgesteuerte Laborschüttler werden in biologischen, medizinischen und pharmazeutischen Laboren für die Kultivierung von Bakterien, Hefe und anderen Organismen in Suspension verwendet. Sie sind z. B. für die Produktion rekombinanter DNA, die Proteinexpression oder das Screening von Kulturen unerlässlich. Da es sich bei einem Laborschüttler in erster Linie um ein gemeinsam genutztes Gerät handelt, das rund um die Uhr bei hohen Drehzahlen und variabler Belastung läuft, muss er langlebig und zuverlässig sein.

Die für einen Benutzer relevanten Kenngrößen für einen Laborschüttler, insbesondere einen Inkubationsschüttler, sind zunächst ein bestimmte Zieltemperatur im Probenlagerraum, eine bestimmte Geschwindigkeit und dazu eine bestimmte Last-Tragfähigkeit der Schüttler-Plattform. Die meisten Anwendungen erfordern die Verwendung unterschiedlicher Gefäßgrößen und -typen: von Platten für das erste Screening über konische Gefäße für Vorkulturen bis hin zu großen Kolben für die Plasmidproduktion oder Proteinexpression. Der ständige Bedarf an höheren Produktausbeuten führte zur Erfindung neuer Kolbentypen, die eine bessere Belüftung als Standard-Schüttelkolben bieten. Dadurch kann das typische Füllvolumen um bis zu 40 % erhöht werden, was zu einem höheren Gewicht auf der Plattform führt. Höhere Drehzahlen über 250 U/min sind für Anwendungen mit z. B. E. coli üblich, um eine signifikante Erhöhung der Zelldichte zu erreichen. Die Anforderungen an Laborschüttler sind deshalb umfangreich, und beinhalten neben der Dauerbelastbarkeit eine ausreichende Versatilität, um insbesondere alle Arten von Plattformkonfigurationen, Lasten und auch hohe Drehzahlen zu bewältigen. Gefordert sind Langlebigkeit und Robustheit für einen zuverlässigen Betrieb über Jahre hinweg.

Als Orbitalschüttler bezeichnet man Laborschüttler, die eine Plattform so bewegen, dass sich alle Punkte auf der Oberseite der Plattform in der X-Y-Ebene auf einer Kreisbahn mit einem gemeinsamen Radius bewegen. Im Allgemeinen werden Bechergläser, Kolben und andere Gefäße an der Oberseite der Plattform befestigt, so dass die darin enthaltene Flüssigkeit an den inneren Seitenwänden des Gefäßes umhergewirbelt wird, um die Durchmischung zu erhöhen und die Wechselwirkung oder den Austausch zwischen der Flüssigkeit und der lokalen gasförmigen Umgebung zu verbessern.

Solche Laborschüttler weisen eine Kammer zur Aufnahme der zu temperierenden Laborproben auf, diese Kammer ist in der Regel innerhalb eines Gehäuses angeordnet. Der Zugang zur Kammer, bei dem der Benutzer die Proben im Gehäuseinneren, insbesondere in der Kammer, lagert und wieder entnimmt, erfolgt in der Regel über eine Gehäuseöffnung, die mittels einer Gehäusetüre verschließbar ist. In weiteren Ausgestaltungen verfügt die Kammer auch über eine Gaszufuhr. Diese Arten von Geräten erlauben die Kultivierung von Zellen in einer CO₂ Atmosphäre. Sie werden Inkubationsschüttler genannt.

Ein bekannter Laborschüttler ist der Innova^{®} S44i, erhältlich von der Eppendorf SE, Hamburg, Deutschland. Er weist einen Auszugsmechanismus auf, mittels dem eine Probenplattform aus der Kammer nach außen gezogen werden kann, wodurch das Beladen und Entladen des Laborschüttlers erheblich vereinfacht ist. Der Auszugsmechanismus weist einen Griff mit Entarretierungstaste auf, der in einer vertikalen Grundstellung arretiert ist. Zum Entarretieren wird der Griff bei gedrückter Entarretierungstaste zunächst gegen die Auszugsrichtung in Richtung der Kammer geführt, wodurch die Arretierungsklinke frei wird. Dann wird der Griff bei gedrückter Arretierungstaste von der Kammer weggezogen und nach unten in eine horizontale Lage gebracht, in der die Probenplattform mithilfe des Griffs und des Auszugsmechanismus nach außen gezogen werden kann. Dieser Sicherungsmechanismus bietet eine hohe Sicherheit für das Sichern der Grundposition der Probenplattform, in der diese vollständig in der Kammer angeordnet ist und in der die Schüttelbewegung des Laborschüttlers vollzogen wird. Der Entsicherungsvorgang wurde aber von Anwendern mitunter als sehr komplex und wenig intuitiv wahrgenommen. Zudem muss die Kammer des genannten Laborschüttler zum Zwecke der Reinigung bzw. des Sterilisierens in mehreren Schritten vorbereitet werden, insbesondere muss der Auszugsmechanismus mithilfe von Werkzeug und ausreichendem Fachwissen bezüglich dieser Schritte demontiert werden. Die Reinigung der Kammer ist insbesondere dann durchzuführen, wenn Kulturflüssigkeit (Zellkulturmedium) ausgelaufen ist, z.B. durch Bruch eines Probengefäßes. In solchen Situationen ist es dringlich, die Kammer möglichst schnell zu räumen, insbesondere auch die Plattform zu entfernen, um die Kammer reinigen zu können. Im Anschluss muss die Kammer wieder befüllt und in Arbeitsbereitschaft versetzt werden. Auch hier ist Schnelligkeit bedeutsam, um möglichst die Probenbewegung bzw. Zellkultivierung ohne große Verzögerung wiederaufzunehmen. Bei unbewegten CO₂ Inkubatoren ist es ein bekanntes Verfahren, die Kammer zusätzlich durch eine Heißtemperaturbehandlung von 180° C über eine gewisse Anzahl an Stunden zu desinfizieren.

Der vorliegenden Erfindung liegt deshalb die Aufgabe zugrunde, einen verbesserten Laborschüttler bereitzustellen, bei dem der Sicherungsmechanismus zum Sichern der Grundposition der Probenplattform einerseits sicher ist und der andererseits einfach und intuitiv zu entsichern ist, insbesondere die Bedienung mit einer einzigen Hand ergonomisch erfolgen kann, ohne größere Kräfte aufwenden zu müssen. Der vorliegenden Erfindung liegt die weitere Aufgabe zugrunde, einen verbesserten Laborschüttler bereitzustellen, dessen Kammer einfacher zu reinigen ist.

Die Erfindung löst diese Aufgabe jeweils durch den Laborschüttler gemäß Anspruch 1, 16 und 19. Weitere technische Lösungen und bevorzugte Ausgestaltungen werden in der Beschreibung genannt, zudem sind weitere bevorzugte Ausgestaltungen Gegenstände der Unteransprüche.

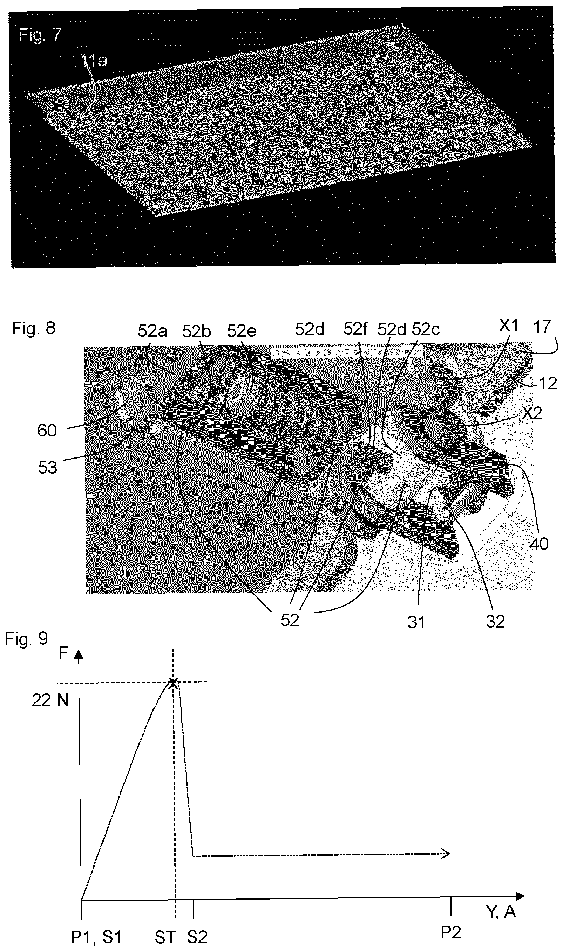

Der Sicherungsmechanismus eines erfindungsgemäßen Laborschüttlers ist einhändig und durch gleichzeitiges Betätigen des Betätigungselements und Ziehen des Griffs in Auszugsrichtung A entsicherbar. Es ergibt sich ein fließender Bewegungsablauf, der von Anwendern als sehr ergonomisch und intuitiv wahrgenommen wird. Zum Entsichern des Sicherungsmechanismus muss insbesondere lediglich mit den Fingern einer Hand das - insbesondere federgelagerte- Betätigungselement gedrückt werden, das insbesondere über einen Zugmechanismus die Entriegelung der Verriegelungseinrichtung, insbesondere des Riegelelements und der Arretierungsöffnung am Trägerelement bewirkt. Gleichzeitig zieht der Anwender am Griff in Auszugsrichtung A, so dass sich der Griff nach unten bewegt; das federgelagerte Betätigungselement kann dabei sofort wieder entlastet werden, was angenehm und intuitiv ist, der Griff rastet insbesondere automatisch und durch die Federspannung bedingt in der zweiten Griffstellung S2 ein, und der Anwender zieht die Trägereinrichtung (inklusive Probenplattform und Gefäßen) mit derselben Auszugsbewegung heraus, mit der er auch den Griff verriegelt hat. Das Sichern in der Auszugsrichtung entgegengesetzter Richtung ist ähnlich einfach und intuitiv. Die vom Anwender aufzubringenden Kräfte sind gering (vgl. Fig. 9).

Die Erfindung bezieht sich auch auf einen Laborschüttler für das Schütteln von in Probengefäßen gelagerten Proben, in der hier auch dargestellten Ausgestaltung, aufweisend:
- eine temperaturregulierbare Kammer mit einer Kammeröffnung,
- eine Trägereinrichtung zum Tragen einer Probenplattform,
- die Probenplattform zum Tragen von Probengefäßen, die auf der Trägereinrichtung angeordnet ist,
- einen in der Kammer befestigten Auszugsmechanismus für den Auszug der Trägereinrichtung, die in einer ersten Position P1 vollständig in der Kammer angeordnet ist, die beim manuellen Ausziehen entlang einer Auszugsrichtung A durch die Kammeröffnung hindurchtritt und die in einer zweiten Position P2 außerhalb der Kammer angeordnet ist, wobei der Auszugsmechanismus eine Basis und mindestens ein Auszugselement aufweist, das entlang der Auszugsrichtung A beweglich an der Basis angeordnet ist, und wobei die Trägereinrichtung an dem mindestens einen Auszugselement befestigt ist, das insbesondere einen Bestandteil eines Schienensystems des Auszugsmechanismus bildet, und das mindestens ein Positionierungselement aufweist, mittels dem die Probenplattform an der Trägereinrichtung positionierbar ist,
- eine Schütteleinrichtung zum Bewegen des Auszugsmechanismus und der Trägereinrichtung in der ersten Position P1,

wobei der Laborschüttler einen Sicherungsmechanismus zum Sichern der ersten Position P1 der Trägereinrichtung aufweist und der Sicherungsmechanismus einen Griff beinhaltet, der an der Trägereinrichtung befestigt ist, der zwischen einer ersten Griffstellung S1 und einer zweiten Griffstellung S2 beweglich ist und der mindestens einen Halteabschnitt, insbesondere ersten Lagerabschnitt, aufweist, und wobei der Sicherungsmechanismus mindestens einen ersten Stopperabschnitt, insbesondere Widerlagerabschnitt, aufweist, der fest mit der Basis verbunden ist,
wobei die Trägereinrichtung in der ersten Position P1 der Trägereinrichtung und in der ersten Griffstellung S1 des Griffs gesichert wird, indem die Probenplattform an dem mindestens einen Positionierelement positioniert ist und zwischen dem mindestens einen Halteabschnitt des Griffs und dem mindestens einen ersten Stopperabschnitt der Basis gehalten wird. Diese Ausgestaltungen des Laborschüttlers kann mit allen anderen hier beschriebenen optionalen Ausgestaltungen des Laborschüttlers kombiniert werden.

Die Erfindung bezieht sich auch auf einen Laborschüttler für das Schütteln von in Probengefäßen gelagerten Proben, in der hier auch dargestellten Ausgestaltung, aufweisend:
- eine temperaturregulierbare Kammer mit einem Kammerboden und einer Kammeröffnung,
- eine Trägereinrichtung zum Tragen einer Probenplattform, auf der die Probengefäße platzierbar sind,
- einen Auszugsmechanismus für den Auszug der Trägereinrichtung, die in einer ersten Position P1 vollständig in der Kammer angeordnet ist, die beim manuellen Ausziehen entlang einer Auszugsrichtung A durch die Kammeröffnung hindurchtritt und die in einer zweiten Position P2 außerhalb der Kammer angeordnet ist,
- eine Schütteleinrichtung insbesondere zum Bewegen des Auszugsmechanismus und der Trägereinrichtung in der ersten Position P1,

wobei der Auszugsmechanismus eine Basis und mindestens ein Auszugselement aufweist, das insbesondere einen Bestandteil eines Schienensystems des Auszugsmechanismus bildet, und das entlang der Auszugsrichtung A beweglich an der Basis angeordnet ist, und wobei die Trägereinrichtung an dem mindestens einen Auszugselement befestigt ist,
wobei der Laborschüttler eine Befestigungseinrichtung aufweist, die mindestens ein, insbesondere am Kammerboden angeordnetes, mit der Schütteleinrichtung verbundenes Befestigungsteil und mindestens ein Verbindungsteil aufweist, mit dem die Basis an dem mindestens einen Befestigungsteil lösbar befestigbar ist, wobei das mindestens eine Befestigungsteil und das mindestens eine Verbindungsteil für eine manuelle und werkzeuglose Befestigung eingerichtet sind. "Werkzeuglos" bedeutet, dass neben der Verbindungseinrichtung, insbesondere außer den beiden Komponenten "Befestigungsteil" und "Verbindungsteil", kein Werkzeug benötigt wird zum Lösen und/oder Herstellen der Verbindung. Insbesondere weist dazu die Verbindungseinrichtung ein integriertes Werkzeug auf, z.B. einen Hebel oder ein Bedienrad, das jeweils unlösbar mit dem Befestigungsteil oder dem Verbindungsteil verbunden ist. Diese Ausgestaltungen des Laborschüttlers kann mit allen anderen hier beschriebenen optionalen Ausgestaltungen des Laborschüttlers kombiniert werden.

Der Laborschüttler zum Schütteln von Laborproben ist insbesondere zum Temperieren der Laborproben eingerichtet. Solche Geräte werden elektrisch betrieben und weisen einen Spannungsanschluss auf. Der Laborschüttler temperiert die Laborproben, das heißt, er hält das Gehäuseinnere und damit die dort lagernden Laborproben im Rahmen von Toleranzen durch eine Temperaturregelung auf einer insbesondere vom Benutzer einstellbaren Solltemperatur. Diese kann über der Raumtemperatur (Umgebungstemperatur) liegen, wie dies bei einem Wärmeschrank oder Inkubator der Fall ist, oder kann unter der Raumtemperatur liegen, wie dies bei einem Kühlschrank oder Gefrierschrank der Fall ist. Bei einem als Klimaborschüttler ausgebildeten Laborschüttler wird vorzugsweise auch ein im Inneren des Gehäuses vorherrschender Klimaparameter im Rahmen von Toleranzen geregelt. Dieser Klimaparameter kann die Luftfeuchtigkeit sein, und/oder eine Gaskonzentration, z.B. eine CO2, O2 und/oder N2-Konzentration. Ein solcher Klimaborschüttler ist beispielsweise ein Laborschüttler zum Schütteln von Laborproben, insbesondere mit lebenden Zellkulturen, mit Inkubatorfunktion, auch bezeichnet als Inkubationsschüttler.

Typische Merkmale solcher Laborschüttler können eine oder mehrere der folgenden Merkmale sein:
Temperaturkontrollierbarkeit der Kammer: Kühlung auf mindestens 4 °C, Beheizung auf maximal 80 °C.
Drehzahlbereich der Schüttelbewegung: (25-500 rpm).
Gehäuseformat derart, dass eine Aufstellbarkeit im Labor (auf dem Labortisch, unter dem Labortisch, stapelbare Standmodelle) möglich ist.
Stapelbarkeit des Gehäuses (2 oder 3 oder mehr übereinander).
Kapazität und Durchsatz: Gefäßtyp, Größe und Kapazität.
Beladungsart (von vorne oder von oben).
CO2-Regelung.
Photosynthetisches Licht.

Besonders bevorzugt ist der Laborschüttler dazu eingerichtet, mithilfe einer Temperiereinrichtung und/oder einer Heizeinrichtung ein Hochtemperaturverfahren im Inneren der Kammer zum Sterilisieren des Kammerinnnenraums durchzuführen, bei dem die Kammer für einen Zeitraum von mehreren Sekunden (z.B. von 1, 2, 5, 10, 30 Sekunden) bis mehreren Minuten (z.B. bis zu 1, 2, 3 5, oder 10 Minuten) einer Temperatur von zwischen 150°C und 200°C, vorzugsweise mindestens 180° C ausgesetzt ist, ohne dass der Auszugsmechanismus, vorzugsweise inklusive der aufgesetzten Probenplattform, entnommen werden muss. Insbesondere ist der Auszugsmechanismus bei der Durchführung des Hochtemperaturzyklus in der verschlossenen Kammer angeordnet. Der Auszugsmechanismus und die Probenplattform sind insbesondere aus entsprechend hochtemperaturbeständigem Material gefertigt, insbesondere einem Edelstahl.

Die Laborschüttler weist vorzugsweise ein Gehäuse auf. Das Gehäuse ist vorzugsweise ein äußeres Gehäuse, dessen Gehäusewände mit der Umgebung in Kontakt stehen. Die Gehäusetüre kann entsprechend eine äußere Gehäusetüre sein, die in der Verschlussposition an die Umgebung grenzt.

Die Gehäusetüre weist insbesondere eine Scharniereinrichtung auf, welche die Gehäusetüre schwenkbar mit dem Gehäuse verbindet. Eine solche Schwenktüre wird durch eine Rotation zwischen einer geöffneten Position und der Verschlussposition bewegt. Die Scharniereinrichtung kann insbesondere an der -im bestimmungsgemäßen Gebrauch des Laborschüttlers - vertikal orientierten Außenkante eines quaderförmigen Gehäuses liegen, welche an die Gehäuseöffnung angrenzt. Die Bodenplatte eines quaderförmigen Gehäuses ist im bestimmungsgemäßen Gebrauch des Laborschüttler horizontal angeordnet, die Seitenwände des Gehäuses sind insbesondere vertikal angeordnet, und die Deckplatte des Gehäuses ist insbesondere der Bodenplatte gegenüberliegend horizontal angeordnet.

Eine Datenverarbeitungseinrichtung ist vorzugsweise Bestandteil der elektrischen Steuereinrichtung, die Funktionen des Laborschüttlers steuert und die der Laborschüttler vorzugsweise aufweist. Die Funktionen der Steuereinrichtung sind insbesondere durch elektronische Schaltkreise implementiert. Die Steuereinrichtung kann einen Mikrocontroller, eine Recheneinheit (CPU) zum Verarbeiten von Daten und/oder einen Mikroprozessor aufweisen, die jeweils die Datenverarbeitungseinrichtung beinhalten können. Die Steuereinrichtung und/oder die Datenverarbeitungseinrichtung ist vorzugsweise zur Durchführung eines Steuerungsverfahrens ausgebildet, das auch als Steuerungssoftware oder Steuerungsprogramm bezeichnet wird. Ein solches Steuerungsverfahren kann den zeitlichen Verlauf einer Schüttelbewegung definieren, die mittels der Schütteleinrichtung durchgeführt werden kann. Diese Schüttelbewegung ist insbesondere definiert durch Richtung(en) von Translationsbewegungen und/oder Amplitude(n) von aufeinanderfolgenden Bewegungsabschnitten, die in einer x-y-Ebene durchgeführt werden. Diese x-y-Ebene ist in der Regel parallel zur Probenplattform und/oder eines Kammerbodens. Bevorzugte Durchmesser einer in einer x-y-Ebene ausgeführten Schüttelbewegung liegen zwischen 0 und 5 cm. Die Schütteleinrichtung, insbesondere ein Orbitalantrieb, ist vorzugsweise für eine Schüttelbewegung mit einem maximalen Durchmesser zwischen 0 und 5 cm eingerichtet. Die Funktionen des Laborschüttlers und/oder der Steuereinrichtung können in Verfahrensschritten beschrieben werden. Sie können als Bestandteile des Steuerungsprogramms realisiert sein, insbesondere als Unterprogramme des Steuerungsprogramms.

Vorzugsweise ist der Laborschüttler ein Inkubationsschüttler. Der Inkubationsschüttler ist dann auch als Labor-Inkubator betreibbar und ist damit ein Gerät, mit dem kontrollierte Klimabedingungen für verschiedene biologische Entwicklungs- und Wachstumsprozesse geschaffen und erhalten werden können. Er dient insbesondere der Schaffung und Erhaltung eines Mikroklimas mit geregelten Gas-, und/oder Luftfeuchtigkeits- und/oder Temperatur-Bedingungen in der Kammer, wobei diese Behandlung zeitabhängig sein kann. Der Inkubationsschüttler kann insbesondere einen Zeitgeber aufweisen, insbesondere eine Zeitschaltuhr, eine als Heiz- und/oder Kühleinrichtung ausgeführte Temperiereinrichtung und vorzugsweise eine Einstellung für die Regelung eines der Kammer zugeführten Austauschgases, eine Einstelleinrichtung für die Zusammensetzung des Gases in der Kammer des Inkubationsschüttler, insbesondere zur Einstellung des CO₂ und/oder des O₂ und/oder des N₂-Gehalts Gehalts des Gases und/oder eine Einstelleinrichtung zur Einstellung der Luftfeuchtigkeit in der Kammer des Inkubationsschüttler.

Der Inkubationsschüttler weist insbesondere die Inkubatorkammer (=Kammer) auf, ferner vorzugsweise eine Regeleinrichtung mit mindestens einem Regelkreis, dem als Stellglied die mindestens eine Temperiereinrichtung und als Messglied mindestens ein Temperatursensor zugeordnet sind. Je nach Ausführungsform kann darüber auch die Luftfeuchte geregelt werden, obwohl die Luftfeuchtigkeit selber nicht durch einen Luftfeuchte-Sensor (rH-Sensor) gemessen wird und die Luftfeuchtigkeit nicht Eingangsgröße des Regelkreises ist. Eine mit Wasser gefüllte Wanne in der Inkubatorkammer kann geheizt oder gekühlt werden, um über die Verdunstung die Luftfeuchtigkeit einzustellen. CO₂- Inkubationsschüttler dienen insbesondere der Kultivierung tierischer bzw. humaner Zellen. Inkubationsschüttler können Wendevorrichtungen zum Wenden des mindestens einen Zellkulturbehälters aufweisen.

Die Steuerungseinrichtung kann dazu eingerichtet sein, dass ein Programmparameter oder ein Steuerungsparameter des Laborschüttlers, insbesondere des Inkubationsschüttlers, automatisch in Abhängigkeit von anderen Daten gewählt wird. Eine von einem Steuerungsparameter gesteuerte Behandlung der mindestens einen Zellkultur in mindestens einem Zellkulturbehälter entspricht bei einem Inkubator insbesondere einer Klimabehandlung, der die mindestens eine Zellkultur unterzogen wird. Mögliche Parameter, insbesondere Programmparameter, insbesondere Nutzerparameter, die zur Beeinflussung einer Klimabehandlung verwendet werden, definieren insbesondere die Temperatur der Kammer, in der die mindestens eine Probe inkubiert wird, die relative Gaskonzentration von O₂- und/oder CO₂ und/oder N₂ in der Kammer, die Luftfeuchtigkeit im in der Kammer und/oder mindestens einen Ablaufparameter, der den Ablauf, insbesondere die Reihenfolge, eines aus mehreren Schritten bestehenden Inkubationsbehandlungsprogramms und/oder Schüttelprogramms beeinflusst oder definiert.

Die Temperiereinrichtung kann eine kombinierte Heiz- / Kühleinrichtung sein. Sie ist vorzugsweise nur eine Heizeinrichtung. Diese kann insbesondere die Wärme über einen elektrischen Widerstandsdraht erzeugen.

Die Laborschüttler bzw. der Inkubationsschüttler kann genau eine Kammer aufweisen, kann aber auch mehrere Kammern aufweisen, deren Atmosphäre (Temperatur, relative Gaskonzentration, Luftfeuchte) insbesondere individuell oder gesammelt einstellbar sein kann. Eine typische Größe des Inneren einer Kammer liegt zwischen 50 und 400 Litern, wobei auch für besondere Anwendungen (IVF) kleinere Kammergrößen möglich sind, insbesondere 10 bis 49 Liter.

Weitere bevorzugte Ausgestaltungen eines erfindungsgemäßen Laborschüttlers lassen sich der Beschreibung der Ausführungsbeispiele gemäß den Figuren entnehmen.

Es zeigen:
Fig. 1a zeigt eine perspektivische seitlich-frontale Ansicht eines erfindungsgemäßen Laborschüttlers gemäß Ausführungsbeispiel, im geschlossenen Zustand der Gehäusetüre.
Fig. 1b zeigt den Laborschüttler aus Fig. 1a, im Zustand mit abgenommener Gehäusetüre und befüllt mit Probengefäßen, mit Griffstellung S1 eines Griffs der in Position P1 angeordneten Trägereinrichtung des Auszugsmechanismus.
Fig. 1c zeigt den Laborschüttler aus Fig. 1b, im Zustand mit Griffstellung S2' des Griffs der in Position P1 angeordneten Trägereinrichtung des Auszugsmechanismus.
Fig. 1d zeigt den Laborschüttler aus Fig. 1b, im Zustand mit Griffstellung S2 des Griffs der in Position P1 angeordneten Trägereinrichtung des Auszugsmechanismus.
Fig. 1e zeigt den Laborschüttler aus Fig. 1d, im Zustand mit teilausgezogener, in Position P2' angeordneter Trägereinrichtung des Auszugsmechanismus.
Fig. 1f zeigt den Laborschüttler aus Fig. 1d, im Zustand mit vollausgezogener, in Position P2 angeordneter Trägereinrichtung des Auszugsmechanismus.
Fig. 2a zeigt den Laborschüttler aus Fig. 1f, ohne Probengefäße und mit von der Trägereinrichtung nach oben abgenommener Probenplattform.
Fig. 2b zeigt den Laborschüttler aus Fig. 2a, mit ausgeblendeter Probenplattform.
Fig. 3a entspricht der Fig. 1f, wobei die Probengefäße von der Probenplattform entfernt wurden.
Fig. 3b entspricht der Fig. 3a, wobei die Auszugsmechanik inklusive deren Basis, Schienensystem, Trägereinrichtung und Probenplattform entfernt wurde, entsprechend dem Zustand nach werkzeugloser Demontage zwecks Reinigung der Kammer.
Fig. 3c zeigt eine Frontansicht der Auszugsmechanik des Laborschüttlers der vorherigen Figuren, bei der insbesondere die Nut-Feder-Führung sichtbar ist, mittels der die translatorische Zwangsführung der Trägereinrichtung bezüglich der Basis gesichert wird.
Fig. 4a zeigt eine perspektivische seitliche Ansicht des Auszugsmechanismus des Laborschüttlers gemäß der vorherigen Figuren, im vollständig eingefahrenen Zustand wie in Position P1.
Fig. 4b zeigt eine seitlich-frontale Aufsicht des Auszugsmechanismus des Laborschüttlers gemäß der vorherigen Figuren, im vollausgezogenen Zustand wie in Position P2.
Fig. 4c zeigt eine seitlich-frontale Untenansicht des Auszugsmechanismus des Laborschüttlers gemäß der vorherigen Figuren, im vollausgezogenen Zustand wie in Position P2.
Fig. 5a zeigt eine seitlich perspektivische Ansicht eines Details der mit Probenplattform versehenen Trägereinrichtung des Laborschüttlers gemäß der vorherigen Figuren, wobei der Sicherungsmechanismus gezeigt ist, mit dem die erste Position P1 der Trägereinrichtung sicherbar ist, mit dem Griff des Sicherungsmechanismus in der senkrechten, ersten Griffstellung S1, und mit der Relativposition R1 des Führungselements bzw. des Verbindungselements relativ zur Zwangsführung.
Fig. 5b zeigt die Ansicht aus Fig. 5a, mit dem Griff des Sicherungsmechanismus in einer Griffstellung S2', und mit der Relativposition R2' des Führungselements bzw. des Verbindungselements relativ zur Zwangsführung.
Fig. 5c zeigt die Ansicht aus Fig. 5a, mit dem Griff des Sicherungsmechanismus in einer horizontalen, zweiten Griffstellung S2, und mit der Relativposition R2 des Führungselements bzw. des Verbindungselements relativ zur Zwangsführung.
Fig. 6a zeigt die Ansicht aus Fig. 5c, mit weiteren Details.
Fig. 6b zeigt die Ansicht aus Fig. 5c, mit weiteren Details.
Fig. 6c zeigt die Ansicht aus Fig. 5a, mit weiteren Details.
Fig. 7 zeigt ein optionales Detail des Laborschüttlers gemäß der vorherigen Figuren, nämlich einen prismenförmigen ersten Stopperabschnitt der Basis des Auszugsmechanismus, und den dazu komplementären prismenförmigen zweiten Stopperabschnitt der Trägereinrichtung, wobei die Trägereinrichtung mit dem zweiten Stopperabschnitt am ersten Stopperabschnitt der Basis in Position P1 anschlägt, wenn die Trägereinrichtung von der Position P1 in die Position P1 bewegt wird.
Fig. 8 zeigt eine seitlich-perspektivische Untenansicht eines Details des Sicherungsmechanismus aus den Figuren 5a bis 5c.
Fig. 9 zeigt eine Weg-Kraft-Kurve, in der die vom Benutzer aufzuwendende Kraft dargestellt ist, wenn der Griff von der ersten Griffstellung in die zweite Griffstellung bewegt wird und die Trägereinrichtung mittels des Griffs in Auszugsrichtung bis in eine Auszugsposition gezogen wird, wobei diese Bewegung eine -abgesehen von rotationsbedingten Komponenten in negative z-Richtung- kontinuierlich in Auszugsrichtung A stattfindet.
Fig. 10a zeigt eine Querschnittansicht entlang einer y-z-Ebene des Laborschüttlers im Zustand der Fig. 1b.
Fig. 10b zeigt eine Querschnittansicht entlang einer y-z-Ebene des Laborschüttlers im Zustand der Fig. 1d.
Fig. 10c zeigt eine Querschnittansicht entlang einer y-z-Ebene des Laborschüttlers im Zustand der Fig. 1f, ohne Probengefäße.

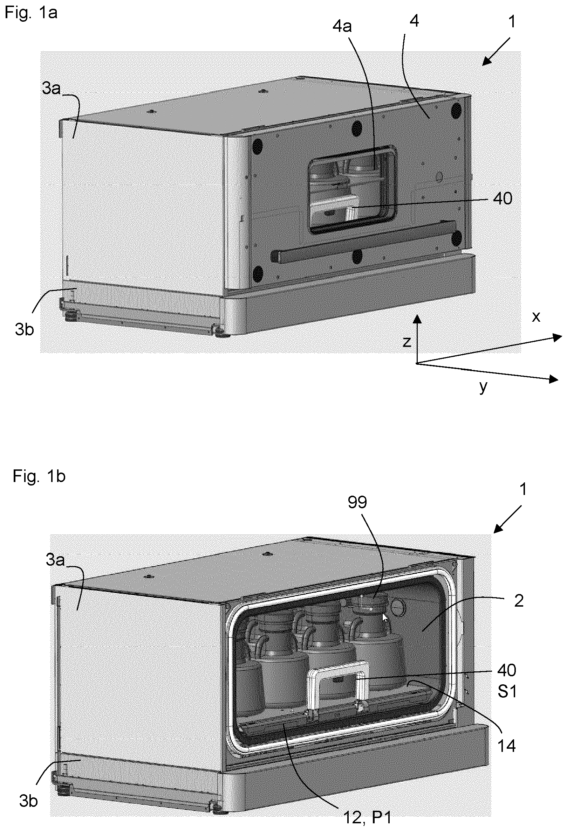

Der in den Figuren dargestellte, Laborschüttler 1 ist in diesem Beispiel ein Inkubationsschüttler, der eine Schüttelbewegung ausführen kann, die von einem hier nicht näher erläuterten Orbitalantrieb erzeugt wird, der unterhalb des Kammerbodens 2a im Bereich des Gehäuseabschnitts 3b angeordnet ist (Fig. 1a). Die zu schüttelnden Proben befinden sich in Laborgefäßen 99, die auf einer Probenplattform 14 platziert sind. Diese kann, insbesondere abnehmbare, Halterungen (nicht gezeigt) für die Probengefäße aufweisen.

Die Probenplattform wird vertikal von oben auf Positionierungsstifte 16 der Trägereinrichtung 12 aufgesetzt, so dass die Probenplattform im wesentlichen unverschiebbar ist, nämlich unverschiebbar innerhalb der x-y-Ebene (im bestimmungsgemäßen Gebrauch des Laborschüttlers die horizontale Ebene, also senkrecht zur Gravitationsrichtung, Fig. 1a), und in negativer z-Richtung auf der Trägereinrichtung oder auf mit dieser fest verbundenen Abschnitten aufliegt.

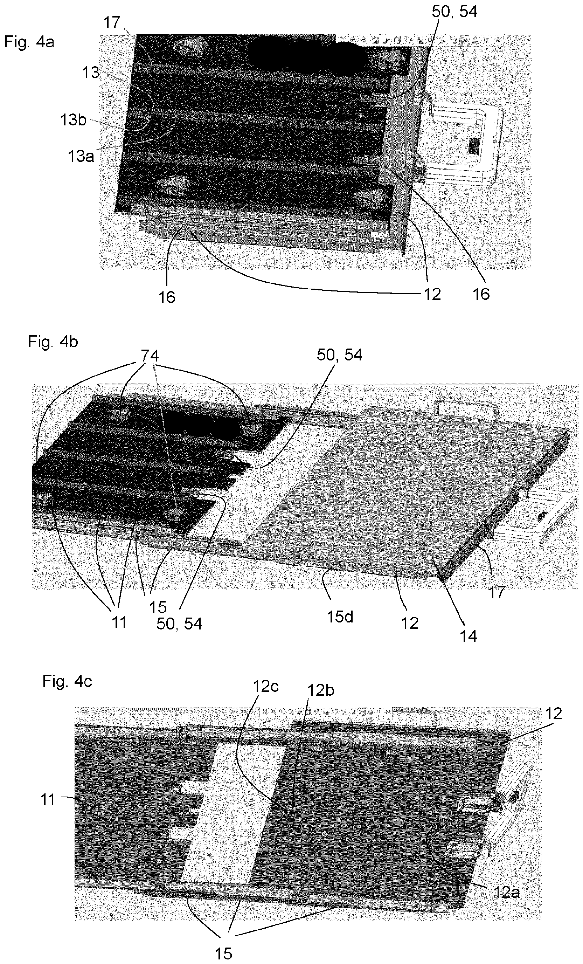

Die Trägereinrichtung ist mittels des Auszugsmechanismus 10, insbesondere dessen Schienensystem, das mindestens eine, hier mehrere Schienen 15 aufweist, translatorisch entlang der Auszugsrichtung A (y im gezeigten kartesischen Koordinatensystem) beweglich. Die maximale Amplitude der translatorischen Beweglichkeit ist durch eine Stoppereinrichtung definiert, die durch Abschnitt der Basis des Auszugsmechanismus 10, insbesondere von Abschnitten der Subplattform 11 des Auszugsmechanismus, festgelegt wird. Ein erster Stopperabschnitt 11a oder 13b (Fig. 4a) ist fest mit der Subplattform 11, also der Basis 11 verbunden. Ein zweiter Stopperabschnitt 12c ist fest mit der Trägereinrichtung verbunden, hier in Form des zweiten Stopperabschnitts 12c, der mit der Unterseite der Trägereinrichtung oder hier vielmehr der Unterseite der mit der Trägereinrichtung 12 verbundenen Probenplattform 14 befestigt ist (Fig. 4c; "nach unten" bezeichnet die Richtung der negativen z-Achse; "nach vorne" bezeichnet die Richtung der positiven y-Achse).

Die Auszugsmechanik ist hier so eingerichtet, dass die Trägereinrichtung 12 in Bezug auf die Basis 11 entlang der z-Achse und entlang der x-Achse jeweils im Wesentlichen unbeweglich ist, und nur translatorisch. Entlang der y-Achse beweglich. Es ist aber auch möglich, dass die Auszugsmechanik ist so eingerichtet ist, dass der Auszug nicht ausschließlich eine translatorische Führung in nur einer Richtung aufweist, sondern z.B. eine gemischt translatorische Auszugsbewegung in unterschiedlichen translatorischen Richtungen ausführt, oder eine rotierende Auszugsbewegung, oder eine gemischt translatorischrotierend verlaufende Auszugsbewegung, z.B. entlang der x-y-Ebene.

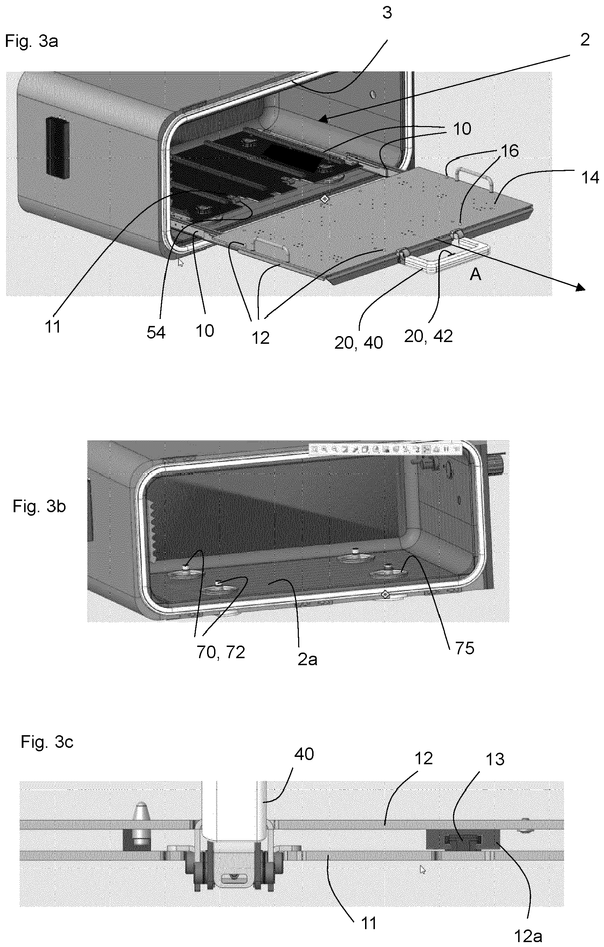

Die Basis des Auszugsmechanismus 10, und damit die daran montierte Probenplattform, ist über eine Befestigungseinrichtung 70, die insbesondere Befestigungselemente 72 (Fig. 3b) bzw. werkzeuglos bedienbare Mutterelementen 74 (Fig. 4b) beinhaltet, die insbesondere ein Feingewinde aufweisen, mit einer Schütteleinrichtung verbunden, insbesondere einem Orbitalantrieb (nicht sichtbar). Dazu sind die Befestigungselemente jeweils innerhalb einer Öffnung 2b des Kammerbodens 2a innerhalb eines Durchmessers von z.B. 5 cm der Öffnung 2b angeordnet und erstrecken sich vertikal vom Bereich unterhalb des Kammerbodens 2a in den Bereich oberhalb des Kammerbodens. Dort weisen die Befestigungselemente 72 Flanschabschnitte zur Auflage der Subplattform 11 der Basis des Auszugsmechanismus auf. Durch diese Anordnung werden Schüttelbewegungen mit Ausschlägen innerhalb des Durchmesserbereichs von z.B. bis zu 5 cm ermöglicht. Es wird die komplette Auszugsmechanik bewegt. Dies ist dadurch begünstigt, dass die Konstruktion des Auszugsmechanismus ausreichend masse-arm ist; insbesondere weist der Auszugsmechanismus bzw. die Schienenelemente 15 Edelstahl auf oder bestehen daraus.

Fig. 1a zeigt eine perspektivische seitlich-frontale Ansicht eines erfindungsgemäßen Laborschüttlers 1 gemäß Ausführungsbeispiel, im geschlossenen Zustand der Gehäusetüre 4, die ein Sichtfenster 4a aufweist, durch die der Griff 40 der Trägereinrichtung 12 und das Innere der Kammer 2 sichtbar sind. Das Gehäuse 3a, 3b des Schüttlers 1 ist im Wesentlichen quaderförmig, ebenso der Kammerinnenraum.

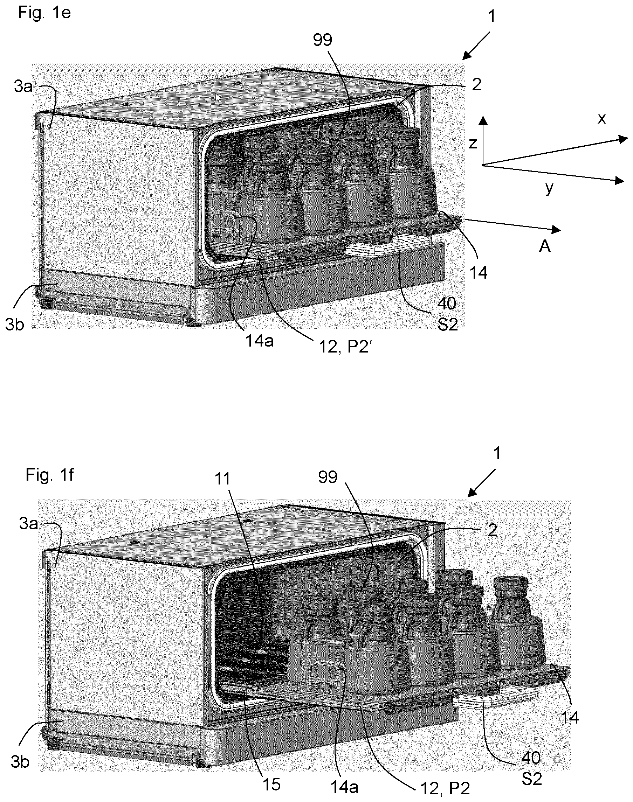

Der Laborschüttler 1 ist eingerichtet für das Schütteln von in Probengefäßen 99 gelagerten Proben. Erweist auf:
- eine temperaturregulierbare Kammer 2 mit einer verschließbaren Kammeröffnung 3,
- eine Trägereinrichtung 12 zum Tragen einer Probenplattform 14, auf der im Bild 1a die Probengefäße 99 platziert sind,
- einen Auszugsmechanismus 10 für den Auszug der Trägereinrichtung 12, die in einer ersten Position P1 (Fig. 1a, 1b) vollständig in der Kammer 2 angeordnet ist, die beim manuellen Ausziehen entlang einer Auszugsrichtung A durch die Kammeröffnung 3 hindurchtritt und die in einer zweiten Position P2 außerhalb der Kammer 2 angeordnet ist, insbesondere vollständig außerhalb der Kammer 2a (Fig. 1f), wenn der Auszug wie hier bevorzugt, ein Vollauszug ist;
- eine Schütteleinrichtung für das Schütteln der Proben 99,
- einen Sicherungsmechanismus 20 zum Sichern und Entsichern der ersten Position P1, mit einer Verriegelungseinrichtung 30 und einem Griff 40, der an der Trägereinrichtung 12 befestigt ist und zwischen einer ersten Griffstellung S1, in der die Verriegelungseinrichtung 30 verriegelt ist, und einer zweiten Griffstellung S2 beweglich ist, in der die Verriegelungseinrichtung 30 entriegelt ist; der Griff ist insbesondere als Schwenkhebel ausgeführt, der hier um eine Achse X1 schwenkbar an der Trägereinrichtung 12 befestigt ist. Die Trägereinrichtung 12 weist vorzugsweise insbesondere Schienenelemente 15 auf, an denen L-förmige Leisten 15d (Fig. 4b) montiert sind, an denen hier die Positionierelemente 16 befestigt sind, und/oder weist vorzugsweise ein Frontprofil 17 (Fig. 4b) auf, an dem der Griff 40 und ein Zwangsführungsabschnitt 60 montiert sind.

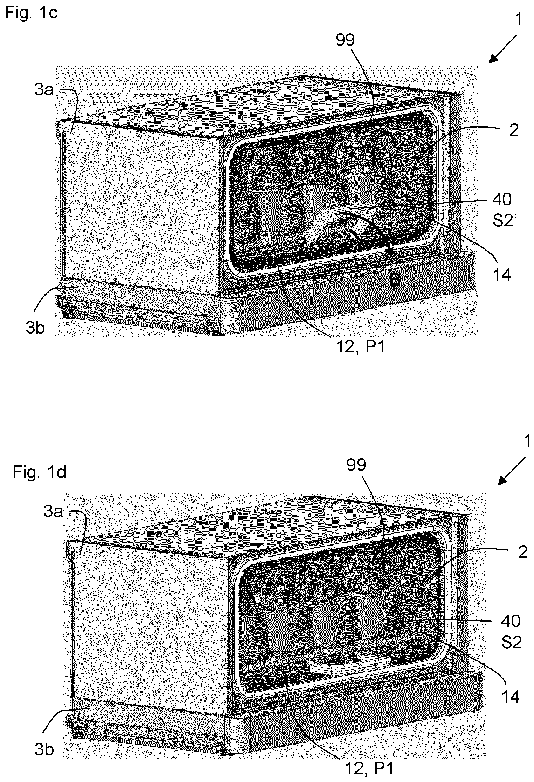

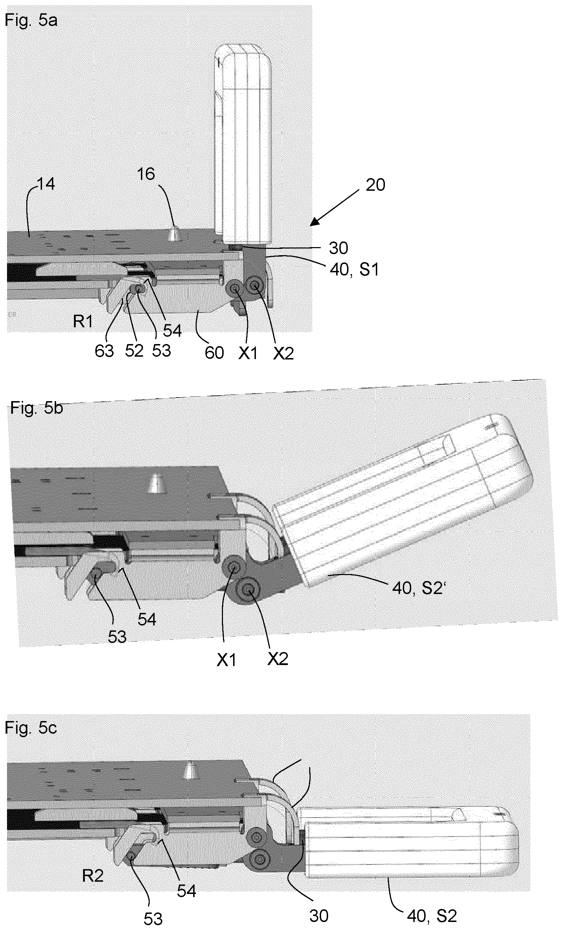

Dabei ist durch die Verriegelungseinrichtung 30 eine Verlagerung der ersten Griffstellung S1 (Fig. 1b, 5a) blockierbar. Die Verriegelungseinrichtung 30 weist einen am Griff 40 angeordnetes, manuell bedienbares Betätigungselement 42 auf, durch dessen Betätigung in Richtung auf den Griff zu, also insbesondere durch einhändige Bedienung, die Verriegelungseinrichtung 30 in der ersten Griffstellung S1 entriegelbar ist, so dass der Griff 40 in die zweite Griffstellung S2 (Fig. 1d, 5c) bewegbar ist, wobei der Sicherungsmechanismus 20 durch gleichzeitiges Betätigen des Betätigungselements 42 und Ziehen des Griffs 40 in Auszugsrichtung A entsicherbar ist. Es ergibt sich ein fließender Bewegungsablauf, der von Anwendern als sehr ergonomisch wahrgenommen wird. Zum Entsichern des Sicherungsmechanismus 20 muss lediglich mit den Fingern einer Hand der federgelagerte Knopf 42 gedrückt werden, der über einen Zugmechanismus die Entriegelung des Riegelelements 31 und der Arretierungsöffnung 32 am Trägerelement bewirkt. Gleichzeitig zieht der Anwender am Griff in Auszugsrichtung, so dass sich der Griff nach unten bewegt; der Knopf 42 kann dabei sofort wieder entlastet werden, was angenehm und intuitiv ist, der Griff rastet automatisch und durch die Federspannung bedingt in der zweiten Griffstellung S2 ein (Fig. 5c), und der Anwender zieht die Trägereinrichtung 12 (inklusive Probenplattform und Gefäßen) mit derselben Auszugsbewegung heraus, mit der er auch den Griff verriegelt hat.

Der Sicherungsmechanismus 20 weist vorzugsweise eine Verbindungseinrichtung 50 auf, die ein, insbesondere mit dem Griff 40 beweglich verbundenes, beweglich angeordnetes Verbindungselement 52 zum Herstellen und Lösen einer lösbaren Verbindung der Trägereinrichtung 12 und einer Basis 11 des Auszugsmechanismus 10 in der ersten Position P1 aufweist. Das Verbindungselement ist mehrteilig und in Fig. 8 ausführlich beschrieben. Der Griff 40 ist vorzugsweise dazu eingerichtet, durch seine schwenkende Bewegung B das Verbindungselement 52 zu bewegen, so dass in einer ersten Griffstellung S1 die lösbare Verbindung der Trägereinrichtung 12 und der Basis 11 des Auszugsmechanismus 10 hergestellt ist und in einer zweiten Griffstellung S2 gelöst ist. Auf diese Weise die Trägereinrichtung 12 an der Basis gesichert. Bevorzugt ist es als alternative oder zusätzliche Ausgestaltung, dass der Riegel 31 der Verriegelungseinrichtung 30 des Griffs 40 in Position P1 des Auszugsmechanismus bzw. der Trägereinrichtung und insbesondere in Griffstellung S2 mit einem Abschnitt 32' verrastet, der fest mit der Kammer bzw. dem Kammerboden verbunden ist. Diese Ausgestaltung ist hier nicht dargestellt.

Vorzugsweise weist die Verbindungseinrichtung mindestens ein Federelement 56 auf, das vorzugsweise so am Griff angeordnet und spannbar ist, dass beim Bewegen des Griffs von der ersten Griffstellung S1 in die zweite Griffstellung S2 durch die manuelle Bedienung des Griffs eine durch das mindestens eine Federelement erzeugte Spannung überwunden werden muss, wenn der Griff zwischen der ersten Griffstellung S1 und zweiten Griffstellung S2 bewegt wird. Dadurch wird die erste Griffstellung S1 gesichert, und aufgrund der Verbindung des Verbindungselements 52 mit der Basis 11 (Haken 54) auch die Grundposition P1 des Auszugsmechanismus gesichert. Diese Ausgestaltung ist hier dargestellt.

Vorzugsweise ist das mindestens eine Federelement 56 so zwischen dem Verbindungselement 12 und dem Griff 40 angeordnet, dass in der ersten Griffstellung S1 die Verbindung der Trägereinrichtung 12 und der Basis 11 durch die Spannung des mindestens einen Federelements 56 gesichert wird. Diese Ausgestaltung ist hier dargestellt.

Vorzugsweise ist das mindestens eine Federelement 56 so zwischen dem Verbindungselement 52 und dem Griff 40 angeordnet, dass mittels dem Federelement 56 eine Totpunktstellung des Griffs eingerichtet ist (wie hier gezeigt), die insbesondere durch die manuelle Bedienung des Griffs überwunden werden muss, wenn dieser zwischen der ersten Griffstellung (S1) und zweiten Griffstellung (S2) bewegt wird. Diese Ausgestaltung ist hier dargestellt.

Dabei ist vorzugsweise durch die Spannung des mindestens einen Federelements 56 der Griff 40 gegen ein Schwenken in die zweite Griffstellung S2 sicherbar, wie hier umgesetzt, indem eine zwischen der ersten S1 und zweiten Griffstellung S2 liegende Totpunktgriffstellung vorgesehen ist, in der insbesondere die Totpunktfeder 56 unter maximaler Spannung steht. Diese Ausgestaltung ist hier dargestellt.

Von einem Totpunkt spricht man, wenn bei einem ebenen Hebelmechanismus die verbindenden Gelenke und die einwirkenden Kraftvektoren auf einer gemeinsamen Geraden liegen. Die Totpunktposition liegt hier insbesondere vor, wenn die Achsen X1, X2 und die Achse des Führungselements 53, betrachtet in der y-z-Ebene, auf einer Geraden liegen. Die entsprechende Griffstellung wird als Totpunktgriffstellung ST bezeichnet.

Dabei wird die zugeführte Kraft nur auf den Haltepunkt des Mechanismus übertragen, eine Bewegung der Hebel ist ohne äußere Einwirkung nicht möglich. Erst eine Kraft quer zur Hauptachse dieses Mechanismus ändert diesen Zustand. Der Totpunkt, der nur gegen eine Federkraft 56 erreichbar und überwindbar ist (z.B. 22 N, siehe Fig. 8) wird zur Sicherung der Grundposition P1 genutzt, es handelt sich um einen selbsthemmenden Mechanismus. Diese Ausgestaltung ist hier dargestellt.

Vorzugsweise ist die Verriegelungseinrichtung 30 in der zweiten Griffstellung S2 verriegelbar ist, so dass durch die Verriegelungseinrichtung 30 eine Verlagerung der zweiten Griffstellung S2 blockierbar ist. Vorzugsweise ist durch die manuelle Betätigung des Betätigungselements 42 die Verriegelungseinrichtung 30 in der zweiten Griffstellung S2 entriegelbar, so dass der Griff 40 in die erste Griffstellung S1 bewegbar ist. Diese Ausgestaltung ist hier dargestellt.

Vorzugsweise ist der Griff (40) um eine erste Schwenkachse (X1) schwenkbar, die insbesondere an der Trägereinrichtung lokalisiert ist. Diese Ausgestaltung ist hier dargestellt.

Vorzugsweise ist das Verbindungselement 52 um eine zweite Schwenkachse X2 schwenkbar, die insbesondere am Griff 40 lokalisiert ist, und die im Wesentlichen parallel, also beabstandet, zur ersten Schwenkachse X1 am Griff 40 angeordnet ist. Diese Ausgestaltung ist hier dargestellt.

Vorzugsweise ist an der Trägereinrichtung 12 ein Zwangsführungsabschnitt 60 mit einer Zwangsführung 63, hier insbesondere eine Kulisse 63, vorgesehen und das Verbindungselement 52, hier insbesondere ein Zuganker mit Feder, weist vorzugsweise mindestens ein Führungselement 53 auf, hier insbesondere eine Kulissenführung 53, das in der ersten Position P1 der Trägereinrichtung 12 von der Zwangsführung 63 führbar ist, wobei das Führungselement 53 in einer ersten Relativposition R1 zur Zwangsführung 63 anordenbar ist, in der die Verbindung des Trägerelements 12 und einer Basis 11 des Auszugsmechanismus 10 hergestellt ist, und in einer zweiten Relativposition zur Zwangsführung anordenbar ist, in der die Verbindung des Trägerelements 12 und der Basis 11 des Auszugsmechanismus 10 gelöst ist, wobei das Führungselement 53 durch die Zwangsführung von der ersten Relativposition in die zweite Relativposition geführt wird, wenn der Griff manuell von der ersten Griffstellung S1 in die zweite Griffstellung (S2) bewegt wird. Auf diese Weise ist der Sicherungsmechanismus 20 komfortabel und intuitiv sowie zuverlässig entsicherbar. Diese Ausgestaltungen sind hier dargestellt.

Vorzugsweise ist die Zwangsführung 63 so eingerichtet ist, dass die Bewegung des Führungselements 53 entlang der Zwangsführung 63 in einer Ebene E verläuft, zu der eine Schwenkachse X1 an einer Basis des Griffs 40 senkrecht verläuft und wobei das Führungselement 53 in der ersten Relativposition R1 in einem entlang einer y-Richtung verlaufenden Endabschnitt 63a der Zwangsführung 63 angeordnet ist und wobei die Zwangsführung 63 vorzugsweise dazu geformt ist, das Führungselement 53 in eine negative z-Richtung zu führen, wenn der Griff 40 von der ersten Griffstellung S1 in die zweite Griffstellung S2 bewegt wird, insbesondere indem die Zwangsführung ausgehend von dem entlang der y-Richtung verlaufenden Endabschnitt schräg nach unten in z-Richtung verläuft, wodurch insbesondere das Verbindungselement (52) von einem Widerlagerelement 54, insbesondere Hakenelement 54, gelöst wird. Diese Ausgestaltungen sind hier dargestellt.

Vorzugsweise ist mit der Basis 11 des Auszugsmechanismus mindestens ein Widerlagerelement 54 fest verbunden, insbesondere ein Hakenelement 54, an dem sich das Verbindungselement 52 in der ersten Griffstellung S1 abstützt, um die Verbindung der Trägereinrichtung mit der Basis 11 des Auszugsmechanismus 10 zu bilden und die translatorische Relativbewegung von Trägereinrichtung 12 und Basis 11 zu verhindern. Diese Ausgestaltungen sind hier dargestellt.

Vorzugsweise stützt sich das Führungselement 53 in der ersten Griffstellung S1 an dem Widerlagerelement 54 ab, insbesondere durch eine formschlüssige Verbindung, die zur Kraftübertragung geeignet ist, insbesondere um die Verbindung der Trägereinrichtung 12 mit der Basis 11 des Auszugsmechanismus 10 zu bilden. Diese Ausgestaltungen sind hier dargestellt.

Vorzugsweise weist der Auszugsmechanismus 10 eine Basis 11 auf, die besonders bevorzugt eine zum Kammerboden 2a und insbesondere zur Probenplattform 14 parallel angeordnete Subplattform 11 aufweist, die zur Montage des Auszugsmechanismus 10 an der Schütteleinrichtung nutzbar ist. Dabei ist vorzugsweise mindestens ein Befestigungsteil 72 vorgesehen, an dem die Basis 11 befestigt ist. Vorzugsweise weist die Kammer 2 einen Kammerboden 2a mit mindestens einer Öffnung 2b auf (hier: 4 Öffnungen 2b). Dabei ist vorzugsweise jeweils ein Befestigungsteil 70 innerhalb einer Öffnung 2b angeordnet und insbesondere mit der Schütteleinrichtung verbunden, so dass eine parallel zum Kammerboden 2a oszillierende Bewegung des Befestigungsteils 72 und insbesondere des damit verbundenen Aufbaus aus Auszugsmechanismus 10 inklusive Trägereinrichtung 12 und Probenplattform 14 ermöglicht. Diese Ausgestaltungen sind hier dargestellt.

Vorzugsweise ist jede der Öffnungen 2b durch einen verformbaren Faltenbalg 75 abgedichtet, der zwischen dem Kammerboden 2a, insbesondere dem Rand der Öffnung 2b und dem Befestigungsteil angeordnet ist. Dadurch wird das Eindringen von Flüssigkeit in den Raum des Antriebs bzw. die Kontamination der Kammer durch Kontaminanten aus dem Antriebsraum verhindert und gleichzeitig die Schüttelbewegung ermöglicht. Diese Ausgestaltungen sind hier dargestellt.

Vorzugsweise weist der Griff mindestens einen Halteabschnitt 43 auf, insbesondere einen ersten Lagerabschnitt 43, wobei der Sicherungsmechanismus 20 vorzugsweise mindestens einen ersten Stopperabschnitt 11a (komplementärer Prismenblock) oder 13b (Anschlagelement), insbesondere einen Widerlagerabschnitt, aufweist, der fest mit der Basis 11 verbunden ist. Dabei wird insbesondere die Trägereinrichtung 12 in der ersten Position P1 der Trägereinrichtung 12 und in der ersten Griffstellung S1 des Griffs 40 gesichert, indem die Probenplattform 14 an dem mindestens einen Positionierelement 16 positioniert ist und zwischen dem mindestens einen Halteabschnitt 43 des Griffs 40 und dem mindestens einen ersten Stopperabschnitt 11a; 13b der Basis 11 gehalten wird, insbesondere durch Kraftbeaufschlagung mit insbesondere der Federkraft 56 oder einer anderen Feder eingeklemmt wird. Auf diese Weise ist die Probenplattform 14 in Position P1 noch besser gegen ein Verschieben in z-Richtung und y-Richtung sicherbar. Diese Ausgestaltungen sind hier größtenteils dargestellt.

Vorzugsweise weist die Kammer 2 einen Kammerboden 2a auf, wobei vorzugsweise der Auszugsmechanismus 10 eine Basis 11 und mindestens ein Auszugselement 15 aufweist, das insbesondere einen Bestandteil eines Schienensystems des Auszugsmechanismus bildet, und das entlang der Auszugsrichtung A beweglich an der Basis 11 angeordnet ist, und wobei die Trägereinrichtung 12 an mindestens einem Auszugselement 15 befestigt ist. Dabei weist der Laborschüttler 1 vorzugsweise eine Befestigungseinrichtung 70 auf, die mindestens ein, insbesondere am Kammerboden 2a angeordnetes, mit der Schütteleinrichtung verbundenes Befestigungsteil 72 und mindestens ein Verbindungsteil 74 aufweist, mit dem die Basis 11 an dem mindestens einen Befestigungsteil 72 lösbar befestigbar ist, wobei das mindestens eine Befestigungsteil 72 und das mindestens eine Verbindungsteil 74 für eine manuelle und werkzeuglose Befestigung eingerichtet sind. Diese Ausgestaltungen sind hier dargestellt. Durch diese manuelle und werkzeuglose Befestigung ergibt sich eine einfache und komfortable Möglichkeit der Reinigung der Kammer, wodurch die Arbeit mit dem Laborschüttler effizient und sicherer wird. Diese Ausgestaltungen sind hier dargestellt. Insbesondere ist das Verbindungsteil 74 dazu eingerichtet, insbesondere mittels eines Feingewindes auf das Befestigungsteil aufschraubbar zu sein. Alternativ und jeweils bevorzugt kann für die werkzeuglose Verbindbarkeit und Lösbarkeit jeweils auch am Befestigungsteil 72 und/oder dem Verbindungsteil 74 verwendet werden: Einfachgewinde, Mehrfachgewinde, Bajonett; Schnellverschluss; Schnellspannmutter. Dadurch lässt sich die Verbindung jeweils komfortabel, einfach, sicher und mit langlebigen Mitteln realisieren.

Die Erfindung bezieht sich auch auf einen Laborschüttler 1 für das Schütteln von in Probengefäßen gelagerten Proben, in der hier auch dargestellten Ausgestaltung, aufweisend:
- eine temperaturregulierbare Kammer 2 mit einer Kammeröffnung 3,
- eine Trägereinrichtung 12 zum Tragen einer Probenplattform 14,
- die Probenplattform 14 zum Tragen von Probengefäßen, die auf der Trägereinrichtung 12 angeordnet ist,
- einen in der Kammer 2 befestigten Auszugsmechanismus 10 für den Auszug der Trägereinrichtung 12, die in einer ersten Position P1 vollständig in der Kammer 2 angeordnet ist, die beim manuellen Ausziehen entlang einer Auszugsrichtung A durch die Kammeröffnung 3 hindurchtritt und die in einer zweiten Position P2 außerhalb der Kammer 2 angeordnet ist, wobei der Auszugsmechanismus 10 eine Basis 11 und mindestens ein Auszugselement 15 aufweist, das entlang der Auszugsrichtung A beweglich an der Basis 11 angeordnet ist, und wobei die Trägereinrichtung 12 an dem mindestens einen Auszugselement 15 befestigt ist, das insbesondere einen Bestandteil eines Schienensystems des Auszugsmechanismus bildet, und das mindestens ein Positionierungselement 16 aufweist, mittels dem die Probenplattform 14 an der Trägereinrichtung 12 positionierbar ist,
- eine Schütteleinrichtung zum Bewegen des Auszugsmechanismus und der Trägereinrichtung (12) in der ersten Position (P1),

wobei der Laborschüttler 1 einen Sicherungsmechanismus 20 zum Sichern der ersten Position P1 der Trägereinrichtung 12 aufweist und der Sicherungsmechanismus 20 einen Griff 40 beinhaltet, der an der Trägereinrichtung 12 befestigt ist, der zwischen einer ersten Griffstellung S1 und einer zweiten Griffstellung S2 beweglich ist und der mindestens einen Halteabschnitt 43, insbesondere ersten Lagerabschnitt 43, aufweist, und wobei der Sicherungsmechanismus 20 mindestens einen ersten Stopperabschnitt 11a; 13b, insbesondere Widerlagerabschnitt 11a; 13b, aufweist, der fest mit der Basis 11 verbunden ist,
wobei die Trägereinrichtung 12 in der ersten Position P1 der Trägereinrichtung 12 und in der ersten Griffstellung S1 des Griffs 40 gesichert wird, indem die Probenplattform 14 an dem mindestens einen Positionierelement 16 positioniert ist und zwischen dem mindestens einen Halteabschnitt 43 des Griffs 40 und dem mindestens einen ersten Stopperabschnitt 11a; 13b der Basis 11 gehalten wird. Diese Ausgestaltungen des Laborschüttlers kann mit allen anderen hier beschriebenen optionalen Ausgestaltungen des Laborschüttlers 1 kombiniert werden. Insbesondere mit folgenden Ausgestaltungen:
   Vorzugsweise wird der mindestens eine Halteabschnitt 43 des Griffs 40 in der ersten Griffstellung S1 durch ein Federelement 56 des Sicherungsmechanismus 20, insbesondere durch eine Totpunktfeder 56, gegen die Probenplattform 14 gedrückt wird und diese wird über mindestens einen, fest mit der Trägereinrichtung verbundenen, zweiten Stopperabschnitt 12c gegen den mindestens einen als Widerlagerabschnitt dienenden zweiten Stopperabschnitt 11a; 13b gedrückt.

Vorzugsweise ist der mindestens eine zweite Stopperabschnitt 11a zur Bildung einer entlang der Auszugsrichtung A beweglichen Nut-Feder-Verbindung (12c; 13) ausgestaltet, und/oder weist einen entlang der Auszugsrichtung A rampenförmigen Verlauf auf, mit dem die Probenplattform 14 in der ersten Position P1 bezüglich einer z-Richtung der Basis 11 festlegbar ist.

Die Erfindung bezieht sich auch auf einen Laborschüttler 1 für das Schütteln von in Probengefäßen gelagerten Proben, in der hier auch dargestellten Ausgestaltung, aufweisend:
- eine temperaturregulierbare Kammer 2 mit einem Kammerboden 2a und einer Kammeröffnung 3,
- eine Trägereinrichtung 12 zum Tragen einer Probenplattform 14, auf der die Probengefäße platzierbar sind,
- einen Auszugsmechanismus 10 für den Auszug der Trägereinrichtung 12, die in einer ersten Position P1 vollständig in der Kammer 2 angeordnet ist, die beim manuellen Ausziehen entlang einer Auszugsrichtung A durch die Kammeröffnung 3 hindurchtritt und die in einer zweiten Position P2 außerhalb der Kammer 2 angeordnet ist,
- eine Schütteleinrichtung insbesondere zum Bewegen des Auszugsmechanismus und der Trägereinrichtung 12 in der ersten Position P1,

wobei der Auszugsmechanismus 10 eine Basis 11 und mindestens ein Auszugselement 15 aufweist, das insbesondere einen Bestandteil eines Schienensystems des Auszugsmechanismus bildet, und das entlang der Auszugsrichtung A beweglich an der Basis 11 angeordnet ist, und wobei die Trägereinrichtung 12 an dem mindestens einen Auszugselement 15 befestigt ist,
wobei der Laborschüttler eine Befestigungseinrichtung 70 aufweist, die mindestens ein, insbesondere am Kammerboden 2a angeordnetes, mit der Schütteleinrichtung verbundenes Befestigungsteil 72 und mindestens ein Verbindungsteil 74 aufweist, mit dem die Basis 11 an dem mindestens einen Befestigungsteil 72 lösbar befestigbar ist, wobei das mindestens eine Befestigungsteil 72 und das mindestens eine Verbindungsteil 74 für eine manuelle und werkzeuglose Befestigung eingerichtet sind. "Werkzeuglos" bedeutet, dass neben der Verbindungseinrichtung, insbesondere außer den beiden Komponenten "Befestigungsteil" und "Verbindungsteil", kein Werkzeug benötigt wird zum Lösen und/oder Herstellen der Verbindung. Insbesondere weist dazu die Verbindungseinrichtung ein integriertes Werkzeug auf, z.B. einen Hebel oder ein Bedienrad, das jeweils unlösbar mit dem Befestigungsteil oder dem Verbindungsteil verbunden ist. Diese Ausgestaltungen des Laborschüttlers kann mit allen anderen hier beschriebenen optionalen Ausgestaltungen des Laborschüttlers 1 kombiniert werden. Insbesondere mit folgenden Ausgestaltungen:
   Vorzugsweise sind jeweils ein Befestigungsteil 72 und ein Verbindungsteil 74 kraftschlüssig und/oder formschlüssig verbindbar, insbesondere verschraubbar, und weisen vorzugsweise ein Feingewinde, Gewinde, Bajonett oder Schnellspanner auf. Vorzugsweise weist die Befestigungseinrichtung 70 mindestens eine Schnellspanneinrichtung zur Befestigung der Basis 11 auf.

Vorzugsweise weist die Kammer einen Kammerboden 2a mit mindestens einer Öffnung 2b, innerhalb der das mindestens eine Befestigungsteil 72 parallel zum Kammerboden 2a beweglich angeordnet ist, insbesondere zur Durchführung einer Orbitalbewegung. Dazu gehören vorzugsweise translatorische Bewegungen; insbesondere sind Translationen so überlagert, dass auf der ganzen Basis an jedem integralen Punkt eine Kreisbewegung abgebildet wird; Ausführung insbesondere gemäß einem Orbitalmischer; dabei gibt ein Exzenter einen Orbit vor und gibt die Amplutude vor; insbesondere wird ein z-Schlag vermieden, es handelt sich vorzugsweise um eine ebene translatorische Bewegung in der x-y-Ebene.

Fig. 1b zeigt den Laborschüttler 1 aus Fig. 1a, im Zustand mit abgenommener Gehäusetüre 4 und befüllt mit Probengefäßen 99, mit Griffstellung S1 eines Griffs der in Position P1 angeordneten Trägereinrichtung des Auszugsmechanismus 10. Fig. 1c zeigt den Laborschüttler 1 aus Fig. 1b, im Zustand mit Griffstellung S2' des Griffs der in Position P1 angeordneten Trägereinrichtung 12 des Auszugsmechanismus 10. Fig. 1d zeigt den Laborschüttler 1 aus Fig. 1b, im Zustand mit Griffstellung S2 des Griffs der in Position P1 angeordneten Trägereinrichtung 12 des Auszugsmechanismus 10. Fig. 1e zeigt den Laborschüttler 1 aus Fig. 1d, im Zustand mit teilausgezogener, in Position P2' angeordneter Trägereinrichtung 12 des Auszugsmechanismus 10. Fig. 1f zeigt den Laborschüttler 1 aus Fig. 1d, im Zustand mit vollausgezogener, in Position P2 angeordneter Trägereinrichtung 12 des Auszugsmechanismus 10.

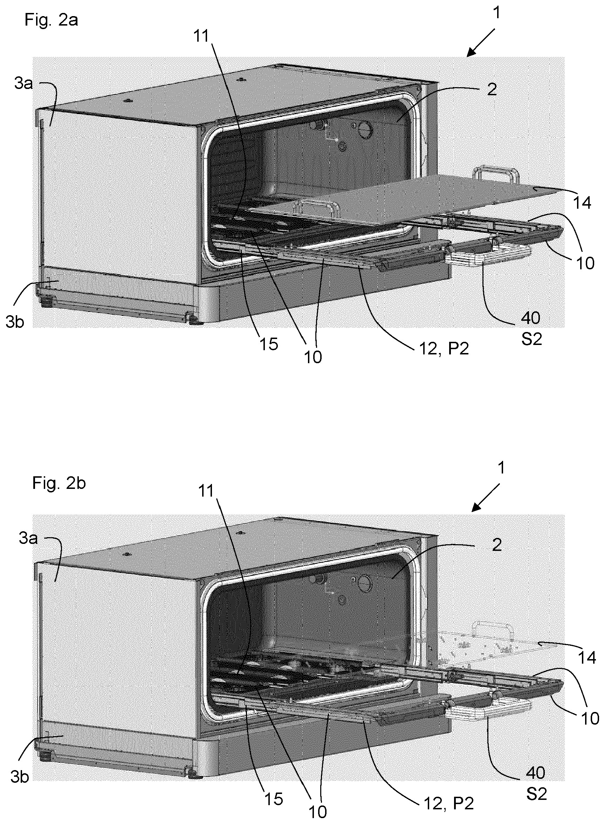

Fig. 2a zeigt den Laborschüttler 1 aus Fig. 1f, ohne Probengefäße 99 und mit von der Trägereinrichtung 12 nach oben (in positive z-Richtung) abgenommener Probenplattform 12. Fig. 2b zeigt den Laborschüttler 1 aus Fig. 2a, mit ausgeblendeter Probenplattform.

Fig. 3a entspricht der Fig. 1f, wobei die Probengefäße 99 von der Probenplattform 14 entfernt wurden. Fig. 3b entspricht der Fig. 3a, wobei die Auszugsmechanik 10 inklusive deren Basis 11, Schienensystem 15, Trägereinrichtung 12 und Probenplattform 14 entfernt wurde, entsprechend dem Zustand nach werkzeugloser Demontage zwecks Reinigung der Kammer. Fig. 3c zeigt eine Frontansicht der Auszugsmechanik 10 des Laborschüttlers 1 der vorherigen Figuren, bei der insbesondere die Nut-Feder-Führung (12c; 13) sichtbar ist, mittels der die translatorische Zwangsführung der Trägereinrichtung 12 bezüglich der Basis 11 gesichert wird.

Fig. 4a zeigt eine perspektivische seitliche Ansicht des Auszugsmechanismus 10 des Laborschüttlers 1 gemäß der vorherigen Figuren, im vollständig eingefahrenen Zustand wie in Position P1. Fig. 4b zeigt eine seitlich-frontale Aufsicht des Auszugsmechanismus 10 des Laborschüttlers 1 gemäß der vorherigen Figuren, im vollausgezogenen Zustand wie in Position P2. Fig. 4c zeigt eine seitlich-frontale Unteransicht des Auszugsmechanismus 10 des Laborschüttlers 1 gemäß der vorherigen Figuren, im vollausgezogenen Zustand wie in Position P2.

Fig. 5a zeigt eine seitlich perspektivische Ansicht eines Details der mit Probenplattform 14 versehenen Trägereinrichtung 12 des Laborschüttlers 1 gemäß der vorherigen Figuren, wobei der Sicherungsmechanismus 20 gezeigt ist, mit dem die erste Position P1 der Trägereinrichtung 12 sicherbar ist, mit dem Griff 40 des Sicherungsmechanismus 20 in der senkrechten, ersten Griffstellung S1, und mit der Relativposition R1 des Führungselements 53 bzw. des Verbindungselements 52 relativ zur Zwangsführung 63. Fig. 5b zeigt die Ansicht aus Fig. 5a, mit dem Griff des Sicherungsmechanismus 20 in einer Griffstellung S2', und mit der Relativposition R2' des Führungselements 53 bzw. des Verbindungselements 51 relativ zur Zwangsführung 63. Fig. 5c zeigt die Ansicht aus Fig. 5a, mit dem Griff des Sicherungsmechanismus in einer horizontalen, zweiten Griffstellung S2, und mit der Relativposition R2 des Führungselements 53 bzw. des Verbindungselements 52 relativ zur Zwangsführung 63.

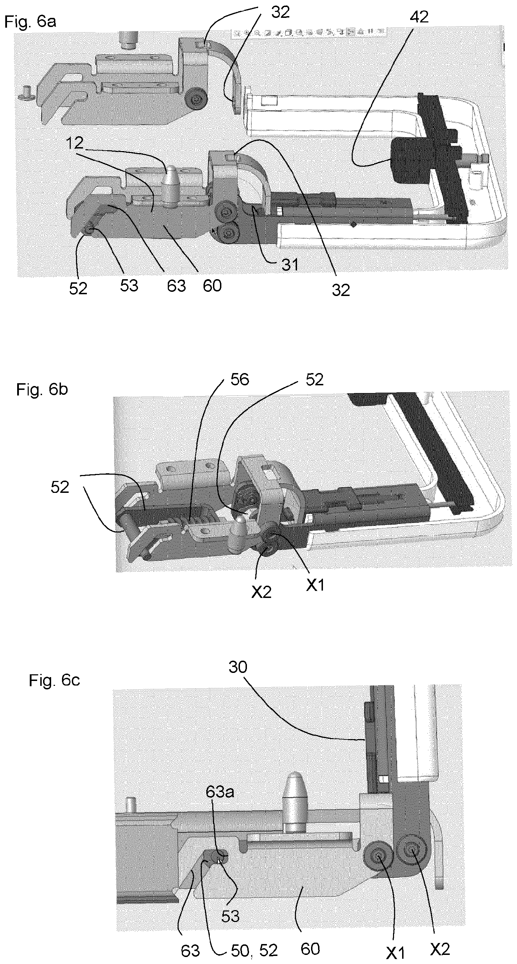

Fig. 6a zeigt die Ansicht aus Fig. 5c, mit weiteren Details. Fig. 6b zeigt die Ansicht aus Fig. 5c, mit weiteren Details. Fig. 6c zeigt die Ansicht aus Fig. 5a, mit weiteren Details.

Fig. 7 zeigt ein optionales Detail des Laborschüttlers 1 gemäß der vorherigen Figuren, nämlich einen prismenförmigen ersten Stopperabschnitt 11a der Basis 11 des Auszugsmechanismus 10, und den dazu komplementären prismenförmigen zweiten Stopperabschnitt der Trägereinrichtung, wobei die Trägereinrichtung 12 mit dem zweiten Stopperabschnitt am ersten Stopperabschnitt 11a der Basis 11 in Position P1 anschlägt, wenn die Trägereinrichtung von der Position P1 in die Position P1 bewegt wird.

Fig. 8 zeigt eine seitlich-perspektivische Untenansicht eines Details des Sicherungsmechanismus aus den Figuren 5a bis 5c.

Fig. 9 zeigt eine Weg-Kraft-Kurve, in der die vom Benutzer aufzuwendende Kraft F dargestellt ist, wenn der Griff unter Passieren der Totpunktgriffstellung ST (gemessen zu Fₘₐₓ= 22N ) von der ersten Griffstellung S1 in die zweite Griffstellung S2 bewegt wird und die Trägereinrichtung 12 mittels des Griffs 40 in Auszugsrichtung A bis in eine Auszugsposition P2 gezogen wird, wobei diese Bewegung eine -abgesehen von rotationsbedingten Komponenten in negative z-Richtung- kontinuierlich in Auszugsrichtung A stattfindet. Vorzugsweise ist der Sicherungsmechanismus und insbesondere das Federelement 56 so eingerichtet, dass die maximale, vom Benutzer beim Ziehen des Griffs aus der Stellung S1 in die Stellung S2 oder insbesondere aus der Stellung S1 bis zur Stellung P2 aufzubringende Auszugskraft Fₘₐₓ kleiner oder gleich ist als vorzugsweise 50N, vorzugsweise 40 N, vorzugsweise 30 N, vorzugsweise 20 N, vorzugsweise 10N.

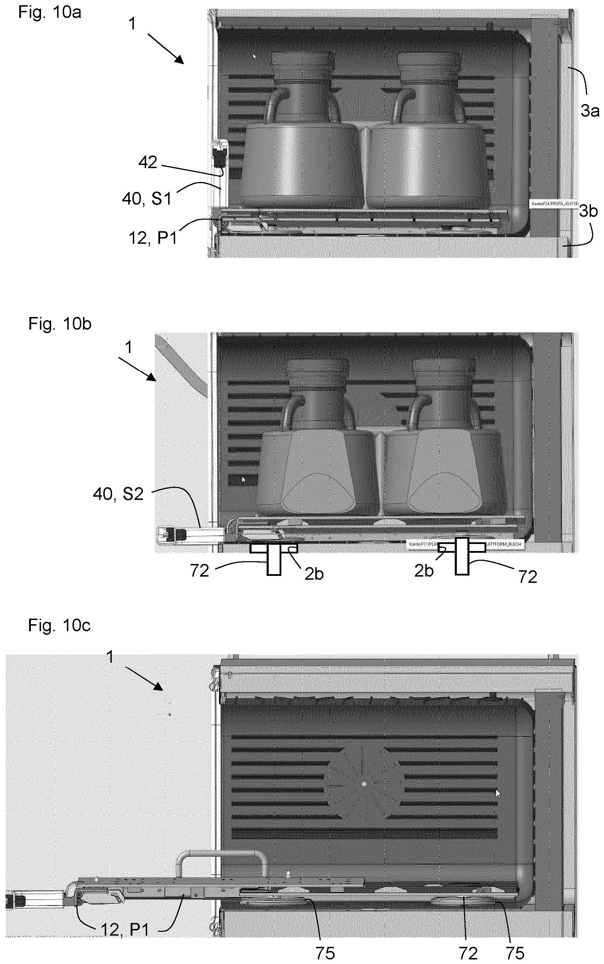

Fig. 10a zeigt eine Querschnittansicht entlang einer y-z-Ebene des Laborschüttlers 1 im Zustand der Fig. 1b. Fig. 10b zeigt eine Querschnittansicht entlang einer y-z-Ebene des Laborschüttlers 1 im Zustand der Fig. 1d. Fig. 10c zeigt eine Querschnittansicht entlang einer y-z-Ebene des Laborschüttlers 1 im Zustand der Fig. 1f, ohne Probengefäße 99.

## Patentansprüche

1. Laborschüttler (1) für das Schütteln von in Probengefäßen gelagerten Proben, aufweisend
eine temperaturregulierbare Kammer (2) mit einer verschließbaren Kammeröffnung (3),
eine Trägereinrichtung (12) zum Tragen einer Probenplattform (14), auf der die Probengefäße platzierbar sind,
einen Auszugsmechanismus (10) für den Auszug der Trägereinrichtung (12), die in einer ersten Position (P1) vollständig in der Kammer angeordnet ist, die beim manuellen Ausziehen entlang einer Auszugsrichtung (A) durch die Kammeröffnung (3) hindurchtritt und die in einer zweiten Position (P2) außerhalb der Kammer (2) angeordnet ist,
eine Schütteleinrichtung für das Schütteln der Proben,
einen Sicherungsmechanismus (20) zum Sichern und Entsichern der ersten Position (P1), mit einer Verriegelungseinrichtung (30) und einem Griff (40), der an der Trägereinrichtung (12) befestigt ist und zwischen einer ersten Griffstellung (S1), in der die Verriegelungseinrichtung (30) verriegelt ist, und einer zweiten Griffstellung (S2) beweglich ist, in der die Verriegelungseinrichtung (30) entriegelt ist,
wobei durch die Verriegelungseinrichtung (30) eine Verlagerung der ersten Griffstellung (S1) blockierbar ist und die Verriegelungseinrichtung (30) ein am Griff (40) angeordnetes, manuell bedienbares Betätigungselement (42) aufweist, durch dessen Betätigung die Verriegelungseinrichtung (30) in der ersten Griffstellung (S1) entriegelbar ist, so dass der Griff (40) in die zweite Griffstellung (S2) bewegbar ist,
und wobei der Sicherungsmechanismus (20) durch gleichzeitiges Betätigen des Betätigungselements (42) und Ziehen des Griffs (40) in Auszugsrichtung (A) entsicherbar ist.

2. Laborschüttler gemäß Anspruch 1, wobei der Sicherungsmechanismus (20) eine Verbindungseinrichtung (50) aufweist, die ein, insbesondere mit dem Griff beweglich verbundenes, beweglich angeordnetes Verbindungselement (52) zum Herstellen und Lösen einer lösbaren Verbindung der Trägereinrichtung (12) und einer Basis (11) des Auszugsmechanismus (10) in der ersten Position (P1) aufweist,
wobei der Griff (40) dazu eingerichtet ist, durch seine Bewegung (B) das Verbindungselement (52) zu bewegen, so dass in einer ersten Griffstellung (S1) die lösbare Verbindung der Trägereinrichtung (12) und der Basis (11) des Auszugsmechanismus (10) hergestellt ist und in einer zweiten Griffstellung (S2) gelöst ist.

3. Laborschüttler gemäß Anspruch 1 oder 2, wobei die Verbindungseinrichtung mindestens ein Federelement (56) aufweist, das vorzugsweise so am Griff angeordnet und spannbar ist, dass beim Bewegen des Griffs von der ersten Griffstellung (S1) in die zweite Griffstellung (S2) durch die manuelle Bedienung des Griffs eine durch das mindestens eine Federelement erzeugte Spannung überwunden werden muss, wenn der Griff zwischen der ersten Griffstellung (S1) und zweiten Griffstellung (S2) bewegt wird.

4. Laborschüttler gemäß Anspruch 3, wobei das mindestens eine Federelement (56) so zwischen dem Verbindungselement und dem Griff angeordnet ist, dass in der ersten Griffstellung (S1) die Verbindung der Trägereinrichtung (12) und der Basis (11) durch die Spannung des mindestens einen Federelements (56) gesichert wird.

5. Laborschüttler gemäß Anspruch 3 oder 4, wobei das mindestens eine Federelement (56) so zwischen dem Verbindungselement und dem Griff angeordnet ist, dass mittels dem Federelement (56) eine Totpunktstellung des Griffs eingerichtet ist, die insbesondere durch die manuelle Bedienung des Griffs überwunden werden muss, wenn dieser zwischen der ersten Griffstellung (S1) und zweiten Griffstellung (S2) bewegt wird,
wobei vorzugsweise durch die Spannung des mindestens einen Federelements (56) der Griff gegen ein Schwenken in die zweite Griffstellung (S2) sicherbar ist, indem eine zwischen der ersten (S1) und zweiten Griffstellung (S2) liegende Totpunktgriffstellung vorgesehen ist, in der insbesondere die Totpunktfeder (56) unter maximaler Spannung steht.

6. Laborschüttler gemäß einem der vorangehenden Ansprüche, wobei die Verriegelungseinrichtung (30) in der zweiten Griffstellung (S2) verriegelbar ist, so dass durch die Verriegelungseinrichtung (30) eine Verlagerung der zweiten Griffstellung (S2) blockierbar ist und wobei durch die manuelle Betätigung des Betätigungselements (42) die Verriegelungseinrichtung (30) in der zweiten Griffstellung (S2) entriegelbar ist, so dass der Griff (4) in die erste Griffstellung (S1) bewegbar ist.

7. Laborschüttler gemäß einem der vorangehenden Ansprüche, wobei der Griff (40) um eine erste Schwenkachse (X1) schwenkbar ist, die insbesondere an der Trägereinrichtung lokalisiert ist, und wobei das Verbindungselement (52) um eine zweite Schwenkachse (X2) schwenkbar ist, die insbesondere am Griff (40) lokalisiert ist, und die echt parallel zur ersten Schwenkachse (X1) am Griff (40) angeordnet ist.

8. Laborschüttler gemäß Anspruch 2 und einem der vorangehenden Ansprüche, wobei an der Trägereinrichtung (12) ein Zwangsführungsabschnitt (60) mit einer Zwangsführung (63) vorgesehen ist und das Verbindungselement (52) ein Führungselement (53) aufweist, das in der ersten Position (P1) der Trägereinrichtung (12) von der Zwangsführung (63) führbar ist, wobei das Führungselement (53) in einer ersten Relativposition (R1) zur Zwangsführung (63) anordenbar ist, in der die Verbindung des Trägerelements (12) und einer Basis (11) des Auszugsmechanismus (10) hergestellt ist, und in einer zweiten Relativposition zur Zwangsführung anordenbar ist, in der die Verbindung des Trägerelements (12) und der Basis (11) des Auszugsmechanismus (10) gelöst ist, wobei das Führungselement (53) durch die Zwangsführung von der ersten Relativposition in die zweite Relativposition geführt wird, wenn der Griff manuell von der ersten Griffstellung (S1) in die zweite Griffstellung (S2) bewegt wird.

9. Laborschüttler gemäß Anspruch 8, dass die Zwangsführung (63) so eingerichtet ist, dass die Bewegung des Führungselements (53) in der Zwangsführung (63) in einer Ebene (E) verläuft, zu der eine Schwenkachse (X1) des Griffs senkrecht verläuft und wobei das Führungselement (53) in der ersten Relativposition (R1) in einem entlang einer y-Richtung verlaufenden Endabschnitt (63a) der Zwangsführung (63) angeordnet ist und wobei die Zwangsführung (63) dazu geformt ist, das Führungselement in eine negative z-Richtung zu führen, wenn der Griff (40) von der ersten Griffstellung (S1) in die zweite Griffstellung (S2) bewegt wird, insbesondere indem die Zwangsführung ausgehend von dem entlang der y-Richtung verlaufenden Endabschnitt schräg nach unten in z-Richtung verläuft, wodurch insbesondere das Verbindungselement (52) von einem Widerlagerelement (54), insbesondere Hakenelement (54), gelöst wird.

10. Laborschüttler gemäß Anspruch 2 und einem der vorangehenden Ansprüche, wobei mit der Basis (11) des Auszugsmechanismus ein Widerlagerelement (54) ortsfest verbunden ist, insbesondere ein Hakenelement (54), an dem sich das Verbindungselement (52) in der ersten Griffstellung (S1) abstützt, um die Verbindung der Trägereinrichtung mit der Basis (11) des Auszugsmechanismus (10) zu bilden und die translatorische Relativbewegung von Trägereinrichtung (12) und Basis (11) zu verhindern.

11. Laborschüttler gemäß Anspruch 10 und Anspruch 8 oder 9, wobei sich das Führungselement (53) in der ersten Griffstellung (S1) an dem Widerlagerelement (54) abstützt, insbesondere durch eine formschlüssige Verbindung, die zur Kraftübertragung geeignet ist, um die Verbindung der Trägereinrichtung mit der Basis (11) des Auszugsmechanismus (10) zu bilden.

12. Laborschüttler gemäß einem der vorangehenden Ansprüche, wobei der Auszugsmechanismus (10) eine Basis (11) aufweist und wobei mindestens ein Befestigungsteil (72) vorgesehen ist, an dem die Basis (11) befestigt ist, und wobei die Kammer (2) einen Kammerboden (2a) mit mindestens einer Öffnung (2b) aufweist, wobei ein Befestigungsteil (70) jeweils innerhalb einer Öffnung (2b) angeordnet und mit der Schütteleinrichtung verbunden ist, so dass eine parallel zum Kammerboden (2a) oszillierende Bewegung des Befestigungsteils ermöglicht ist.

13. Laborschüttler gemäß einem der vorangehenden Ansprüche, wobei jede der Öffnungen (2b) durch einen verformbaren Faltenbalg (75) abgedichtet ist, der zwischen dem Kammerboden (2a), insbesondere dem Rand der Öffnung (2b) und dem Befestigungsteil angeordnet ist.

14. Laborschüttler gemäß Anspruch 1, aufweisend die Probenplattform (14), wobei der Griff mindestens einen Halteabschnitt (43), insbesondere ersten Lagerabschnitt (43), aufweist, und wobei der Sicherungsmechanismus (20) mindestens einen ersten Stopperabschnitt (11a; 13b), insbesondere Widerlagerabschnitt (11a; 13b), aufweist, der fest mit der Basis (11) verbunden ist,
wobei die Trägereinrichtung (12) in der ersten Position (P1) der Trägereinrichtung (12) und in der ersten Griffstellung (S1) des Griffs (40) gesichert wird, indem die Probenplattform (14) an dem mindestens einen Positionierelement (16) positioniert ist und zwischen dem mindestens einen Halteabschnitt (43) des Griffs (40) und dem mindestens einen ersten Stopperabschnitt (11a; 13b) der Basis (11) gehalten wird.

15. Laborschüttler gemäß Anspruch 1, wobei die Kammer (2) einen Kammerboden (2a) aufweist und wobei der Auszugsmechanismus (10) eine Basis (11) und mindestens ein Auszugselement (15) aufweist, das insbesondere einen Bestandteil eines Schienensystems des Auszugsmechanismus bildet, und das entlang der Auszugsrichtung (A) beweglich an der Basis (11) angeordnet ist, und wobei die Trägereinrichtung (12) an mindestens einem Auszugselement (15) befestigt ist,
wobei der Laborschüttler eine Befestigungseinrichtung (70) aufweist, die mindestens ein, insbesondere am Kammerboden (2a) angeordnetes, mit der Schütteleinrichtung verbundenes Befestigungsteil (72) und mindestens ein Verbindungsteil (74) aufweist, mit dem die Basis (11) an dem mindestens einen Befestigungsteil (72) lösbar befestigbar ist, wobei das mindestens eine Befestigungsteil (72) und das mindestens eine Verbindungsteil (74) für eine manuelle und werkzeuglose Befestigung eingerichtet sind.

16. Laborschüttler für das Schütteln von in Probengefäßen gelagerten Proben, aufweisend
eine temperaturregulierbare Kammer (2) mit einer Kammeröffnung (3),
eine Trägereinrichtung (12) zum Tragen einer Probenplattform (14),
die Probenplattform (14) zum Tragen von Probengefäßen, die auf der Trägereinrichtung (12) angeordnet ist,
einen in der Kammer (2) befestigten Auszugsmechanismus (10) für den Auszug der Trägereinrichtung (12), die in einer ersten Position (P1) vollständig in der Kammer (2) angeordnet ist, die beim manuellen Ausziehen entlang einer Auszugsrichtung (A) durch die Kammeröffnung (3) hindurchtritt und die in einer zweiten Position (P2) außerhalb der Kammer (2) angeordnet ist,
wobei der Auszugsmechanismus (10) eine Basis (11) und mindestens ein Auszugselement (15) aufweist, das entlang der Auszugsrichtung (A) beweglich an der Basis (11) angeordnet ist, und wobei die Trägereinrichtung (12) an dem mindestens einen Auszugselement (15) befestigt ist, das insbesondere einen Bestandteil eines Schienensystems des Auszugsmechanismus bildet, und das mindestens ein Positionierungselement (16) aufweist, mittels dem die Probenplattform (14) an der Trägereinrichtung (12) positionierbar ist,
eine Schütteleinrichtung zum Bewegen des Auszugsmechanismus und der Trägereinrichtung (12) in der ersten Position (P1),
wobei der Laborschüttler (1) einen Sicherungsmechanismus (20) zum Sichern der ersten Position (P1) der Trägereinrichtung (12) aufweist und der Sicherungsmechanismus (20) einen Griff (40) beinhaltet, der an der Trägereinrichtung (12) befestigt ist, der zwischen einer ersten Griffstellung (S1) und einer zweiten Griffstellung (S2) beweglich ist und der mindestens einen Halteabschnitt (43), insbesondere ersten Lagerabschnitt (43), aufweist, und wobei der Sicherungsmechanismus (20) mindestens einen ersten Stopperabschnitt (11a; 13b), insbesondere Widerlagerabschnitt (11a; 13b), aufweist, der fest mit der Basis (11) verbunden ist,
wobei die Trägereinrichtung (12) in der ersten Position (P1) der Trägereinrichtung (12) und in der ersten Griffstellung (S1) des Griffs (40) gesichert wird, indem die Probenplattform (14) an dem mindestens einen Positionierelement (16) positioniert ist und zwischen dem mindestens einen Halteabschnitt (43) des Griffs (40) und dem mindestens einen ersten Stopperabschnitt (11a; 13b) der Basis (11) gehalten wird.

17. Laborschüttler gemäß Anspruch 16, wobei der mindestens eine Halteabschnitt (43) des Griffs (40) in der ersten Griffstellung (S1) durch ein Federelement (56) des Sicherungsmechanismus (20), insbesondere durch eine Totpunktfeder (56), gegen die Probenplattform (14) gedrückt wird und diese über mindestens einen, fest mit der Trägereinrichtung verbundenen, zweiten Stopperabschnitt (12c) gegen den mindestens als Widerlagerabschnitt dienenden zweiten Stopperabschnitt (11a; 13b) drückt.

18. Laborschüttler gemäß Anspruch 16 oder 17, wobei der mindestens eine zweite Stopperabschnitt (11a) zur Bildung einer entlang der Auszugsrichtung (A) beweglichen Nut-Feder-Verbindung ausgestaltet ist, und/oder einen entlang der Auszugsrichtung rampenförmigen Verlauf aufweist, mit dem die Probenplattform in der ersten Position bezüglich einer z-Richtung der Basis festlegbar ist.

19. Laborschüttler für das Schütteln von in Probengefäßen gelagerten Proben, aufweisend
eine temperaturregulierbare Kammer (2) mit einem Kammerboden (2a) und einer Kammeröffnung (3),
eine Trägereinrichtung (12) zum Tragen einer Probenplattform (14), auf der die Probengefäße platzierbar sind,
einen Auszugsmechanismus (10) für den Auszug der Trägereinrichtung (12), die in einer ersten Position (P1) vollständig in der Kammer (2) angeordnet ist, die beim manuellen Ausziehen entlang einer Auszugsrichtung (A) durch die Kammeröffnung (3) hindurchtritt und die in einer zweiten Position (P2) außerhalb der Kammer (2) angeordnet ist,
eine Schütteleinrichtung zum Bewegen des Auszugsmechanismus und der Trägereinrichtung (12) in der ersten Position (P1),
wobei der Auszugsmechanismus (10) eine Basis (11) und mindestens ein Auszugselement (15) aufweist, das insbesondere einen Bestandteil eines Schienensystems des Auszugsmechanismus bildet, und das entlang der Auszugsrichtung (A) beweglich an der Basis (11) angeordnet ist, und wobei die Trägereinrichtung (12) an dem mindestens einen Auszugselement (15) befestigt ist,
wobei der Laborschüttler eine Befestigungseinrichtung (70) aufweist, die mindestens ein, insbesondere am Kammerboden (2a) angeordnetes, mit der Schütteleinrichtung verbundenes Befestigungsteil (72) und mindestens ein Verbindungsteil (74) aufweist, mit dem die Basis (11) an dem mindestens einen Befestigungsteil (72) lösbar befestigbar ist, wobei das mindestens eine Befestigungsteil (72) und das mindestens eine Verbindungsteil (74) für eine manuelle und werkzeuglose Befestigung eingerichtet sind.

20. Laborschüttler gemäß Anspruch 19, wobei jeweils ein Befestigungsteil (72) und ein Verbindungsteil (74) verschraubbar sind und ein Feingewinde aufweisen, oder wobei die Befestigungseinrichtung (70) mindestens eine Schnellspanneinrichtung zur Befestigung der Basis (11) aufweist.

21. Laborschüttler gemäß einem der vorangehenden Ansprüche, wobei die Kammer einen Kammerboden (2a) mit mindestens einer Öffnung (2b noch nicht gezeichnet) aufweist, innerhalb der das mindestens eine Befestigungsteil (72) parallel zum Kammerboden (2a) oszillierbar beweglich angeordnet ist.
